# EUROPÄISCHE PATENTSCHRIFT

(11) **EP 1 476 098 B1**
(45) Veröffentlichungstag und Bekanntmachung des Hinweises auf die Patenterteilung: **02.04.2008**
(21) Anmeldenummer: 02710746.5
(22) Anmeldetag: 19.02.2002
(51) Int. Cl.: A61F 2/44

(54) **ZWISCHENWIRBELIMPLANTAT**
INTERVERTEBRAL IMPLANT
IMPLANT INTERVERTEBRAL

(43) Veröffentlichungstag der Anmeldung: 17.11.2004
(73) Patentinhaber: Synthes GmbH, 4436 Oberdorf (CH)
(72) Erfinder: MATHIEU, Claude, CH-2544 Bettlach (CH); CAIN, Chris, M., J., Norwood, SA 5067 (AU)
(74) Vertreter: Lusuardi, Werther
(86) Internationale Anmeldenummer: PCT/CH2002/000099
(87) Internationale Veröffentlichungsnummer: WO 2003/070128

(56) Entgegenhaltungen:
- WO-A-00/66045
- WO-A-01/95837
- WO-A-99/38463
- US-A- 5 776 194
- US-A1- 2002 010 511

## Beschreibung

Die Erfindung betrifft ein Zwischenwirbelimplantat, gemäss dem Oberbegriff des Patentanspruchs 1.

Aus der GB-A-2 207 607 ist ein derartiges Zwischenwirbelimplantat bekannt, welches eine hufeisenförmige Gestalt aufweist mit einer Mehrzahl von zylindrischen Löchern. Die Löcher sind innen glatt ausgebildet und weisen lediglich einen Anschlag für die Köpfe der darin einzuführenden Knochenschrauben auf. Die Nachteile dieser Anordnung bestehen darin, dass die darin eingeführten Befestigungsschrauben nur mit ihrem Schaft im Knochen verankert werden können, ohne dass eine rigide Verbindung mit dem hufeisenförmigen Zwischenwirbelimplantat resultiert. Sobald es zu einer Schwächung der Verankerung des Schraubenschaftes im Knochen kommt, wird das Zwischenwirbelimplantat beweglich gegenüber der Schraube und es besteht die Tendenz einer Migration der Knochenschrauben unter Gefährdung der Blutgefässe. Die Lockerung des Zwischenwirbelimplantates kann zudem zu einer Pseudoarthrose führen.

Ein weiteres ähnliches Zwischenwirbelimplantat ist aus der US-A 2002/010511 bekannt, welches eine Mehrzahl den Körper schräg durchdringender Bohrungen zur Aufnahme von Fixationselementen umfasst.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung oder ihrer Priorität auch tatsächlich publiziert oder öffentlich bekannt war.

Hier will die Erfindung Abhilfe schaffen. Der Erfindung liegt das Problem zugrunde, ein Zwischenwirbelimplantat zu schaffen, welches aus einem körperverträglichen Material besteht und welches durch zusätzliche Mittel eine rigide Verbindung zwischen den Fixationselementen und dem Zwischenwirbelimplantat gestattet, so dass auch bei einer Schwächung der Knochenstruktur keine Lockerung zwischen Zwischenwirbelimplantat und Knochenfixationsmitteln auftritt.

Die Erfindung löst die gestellte Aufgabe mit einem Zwischenwirbelimplantat, welches die Merkmale des Anspruchs 1 aufweist.

Die durch die Erfindung erreichten Vorteile ergeben sich im wesentlichen durch die rigide, d.h. feste Verbindung zwischen dem Zwischenwirbelimplantat und den longitudinalen Fixationselementen mittels einer Frontplatte.

Bei der erfindungsgemässen Ausführung ist an der Vorderfläche des dreidimensionalen Körpers eine zur horizontalen Mittelebene des Zwischenwirbelimplantates vertikal angeordnete Frontplatte angebracht, durch welche Bohrungen hindurchgehen und in welcher longitudinale Fixationselemente verankerbar sind. Dies hat gegenüber zweiteiligen Implantaten gemäss dem Stand der Technik, bei welchen in einem gesonderten Operationsschritt eine Frontplatte implantiert wird, den Vorteil, dass die Implantation des Zwischenwirbelimplantates einschrittig und damit einfacher und schneller durchführbar ist. Ein weiterer Vorteil liegt darin begründet, das dadurch die Fixation des Zwischenwirbelimplantates möglichst frontal am Wirbelkörper erfolgt, d.h. dort wo in der Regel gutes Knochenmaterial vorhanden ist. Das Resultat ist eine anteriore Bewegungseinschränkung, ohne dass dadurch ein grösseres Risiko für die umliegenden Strukturen entsteht als dies mit einem Zwischenwirbelimplantat gemäss dem Stand der Technik der Fall ist. Die Last wird vom Zwischenwirbelimplantat unter Kompression immer noch aufgenommen und nicht durch die Frontplatte oder die Fixationsschrauben.

Bei einer besonderen Ausführungsform ist die Frontplatte im dreidimensionalen Körper verschieblich angeordnet, so dass sie sich gegenüber dem dreidimensionalen Körper in der Vertikalen verschieben kann. Dadurch erreicht man ein "stress shielding" (Schutz, bzw. Neutralisierung von mechanischen Spannungen), welches eine graduelle Anpassung der Endplatten an das Zwischenwirbelimplantat während des Heilungsprozesses erlaubt.

Bei einer weiteren Ausführungsform ist die Frontplatte aus einem anderen Material gefertigt als der dreidimensionale Körper.

Bei einer weiteren Ausgestaltung verjüngt sich mindestens eine Bohrung gegen die Unterseite hin konusförmig, so dass eine Knochenschraube mit korrespondierendem Konuskopf darin rigide verankert werden kann. Die konische Bohrung besitzt vorzugsweise einen Konuswinkel, der kleiner als der resultierende Reibungswinkel ist. Zweckmässigerweise beträgt die Konizität der konischen Bohrung 1 : 3,75 bis 1 : 20,00, vorzugsweise 1 : 5 bis 1 : 15.

Bei einer weiteren Ausführungsform sind die Seitenflächen des Zwischenwirbelimplantats im wesentlichen alle konvex ausgebildet.

Zweckmässigerweise sind die Oberseite und/oder die Unterseite des Zwischenwirbelimplantats nicht planar, sondern vorzugsweise konvex ausgebildet. Damit kann eine bessere Anpassung an die Endplatten der benachbarten Wirbelkörper erreicht werden.

Bevorzugt durchbohren die Bohrungen nicht die linke und die rechte Seitenfläche des Zwischenwirbelimplantats. Auch die Vorderfläche wird vorteilhafterweise nicht von den Bohrungen durchquert.

Bei einer weiteren Ausführungsform verlaufen mindestens zwei der Bohrungen parallel. Dadurch wird die Einführbarkeit des Zwischenwirbelimplantats bei der Implantation erleichtert.

Bei einer anderen Ausführungsform verlaufen mindestens zwei der Bohrungen von der Vorderseite aus betrachtet divergent. Dadurch gelangt man mit den Knochenschrauben in einen Bereich des Wirbelkörpers, der im Vergleich zu dessen Zentrum eine bessere Knochenqualität aufweist.
Zweckmässigerweise weisen die Achsen der Bohrungen relativ zur horizontalen Mittelebene einen Winkel im Bereich von 25° bis 70°, vorzugsweise von 35° bis 55° auf. Damit wird ein besserer Zugang bei der Einbringung der Schrauben erreicht.

Bei einer weiteren Ausführungsform wird die horizontale Mittelebene von den Bohrungen nicht durchstossen.

Je nach den Umständen können zwei, drei, vier oder auch mehr longitudinale Fixationselemente in rigider Weise mit dem Zwischenwirbelimplantat verbunden werden, zweckmässigerweise sollte mindestens ein Fixationselement die Oberseite und mindestens ein Fixationselement die Unterseite des Zwischenwirbelimplantats durchstossen.

Vorzugsweise werden longitudinale Fixationselemente in Form von Knochenschrauben mit einem Kopf und einem Schaft verwendet, wobei der Kopf vorzugsweise mit einem Aussengewinde versehen ist, welches mit dem Innengewinde der Bohrung des Zwischenwirbelimplantats korrespondiert. Bei einer zweiten möglichen rigiden Verbindungsart kann vorzugsweise eine Knochenschraube verwendet werden, bei welcher sich der Kopf gegen den Schaft hin konisch verjüngt, wobei die Konizität des Kopfes der Konizität der Bohrung des Zwischenwirbelimplantates entspricht.

Bei einer weiteren Ausführungsform durchstossen mindestens zwei longitudinale Fixationselemente die Oberseite und mindestens zwei longitudinale Fixationselemente die Unterseite. Damit erhält das Zwischenwirbelimplantat eine optimale Verankerung in den benachbarten Wirbelkörpern.

Die als Knochenschrauben ausgebildeten, longitudinalen Fixationselemente weisen vorzugsweise ein selbstbohrendes und selbstschneidendes Aussengewinde auf. Die longitudinalen Fixationselemente können auch als gewindelose Zylinderstifte ausgebildet sein, weiche mit einer Bohrerspitze, vorzugsweise in Form eines Trokars versehen sind.

Eine weitere Variante besteht darin, dass die longitudinalen Fixationselemente als Spiralfedern ausgebildet sind und schliesslich können die longitudinalen Fixationselemente auch als ein- oder mehrflügelige Spiralklingen ausgebildet sein.

Bei einer weiteren Ausführungsform ist die Spitze des longitudinalen Fixationselementes im Körper des Zwischenwirbelimplantates verankerbar, so dass der Kopf des longitudinalen Fixationselementes im benachbarten Wirbelkörper verankerbar ist.

Bei einer weiteren Ausführungsform weist der Kopf des longitudinalen Fixationselementes einen erweiterten Durchmesser auf und zudem ist zusätzlich eine Unterlagsscheibe für diesen Kopf vorgesehen, welche zur Anlage an den Wirbelkörper bestimmt ist.

Das Zwischenwirbelimplantat kann aus irgendeinem körperverträglichen Material gefertigt werden, zweckmässigerweise besteht jedoch der Körper aus einem körperverträglichen Kunststoff, vorzugsweise einem unverstärkten Kunststoff. Der Vorteil gegenüber den in der Implantologie bereits bekannten faserverstärkten Kunststoffen besteht darin, dass keine Verstärkungsfasern freigelegt werden, was klinisch nachteilig ist. In einen solchen aus unverstärktem Kunststoff bestehenden Körper, können zweckmässigerweise Knochenschrauben verwendet werden, deren Aussengewinde einen Lastflankenwinkel im Bereich von 11° bis 14°, vorzugsweise von 12° bis 13° aufweist. Die vergleichsweise geringe Neigung der Lastflanke bewirkt eine hohe Klemmkraft, wodurch die Radialdehnung und die Rissgefahr im Kunststoff reduziert wird. Zweckmässigerweise weist das Aussengewinde der Knochenschrauben einen Steigungswinkel im Bereich von 6° bis 10°, vorzugsweise von 7° bis 9° auf. Dieser spezielle Steigungswinkel erzeugt eine Selbsthemmung im Gewinde und sichert damit die Knochenschraube gegen ein selbständiges Lösen.

Um die Verankerung der Knochenschraube im Kunststoff-Körper zu verbessern, kann die Bohrung eine Metallhülse mit Innengewinde sein. Das Zwischenwirbelimplantat kann auch nur teilweise aus einem Kunststoff und - im Bereich der Bohrungen - aus Metall bestehen. Damit wird insgesamt eine bessere Führung und Verankerung der Knochenschrauben im Zwischenwirbelimplantat erreicht.

Bei einer weiteren Ausführungsform können die Bohrungen eine glatte Innenwand aufweisen, in welche der Gewindekopf eines metallischen, longitudinalen Fixationselementes eingeschnitten oder eingeformt werden kann.

Die Erfindung und Weiterbildungen der Erfindung werden im folgenden anhand der teilweise schematischen Darstellung zweier Ausführungsbeispiele noch näher erläutert.

Es zeigen:
Fig. 1 eine perspektivische Ansicht mit teilweisem Schnitt des Zwischenwirbelimplantates mit eingesetzten Knochenschrauben, gemäss dem Stand der Technik;
Fig. 2 eine Vorderansicht des Zwischenwirbelimplantates nach Fig. 1;
Fig. 3 eine Seitenansicht des Zwischenwirbelimplantates nach Fig. 1;
Fig. 4 eine Aufsicht auf das Zwischenwirbelimplantat nach Fig. 1;
Fig. 5 eine Vorderansicht einer Ausführungsform des erfindungsgemässen Zwischenwirbelimplantates mit einem Vordereinsatz in teilweiser Schnittdarstellung;
Fig. 6 einen vertikalen Längsschnitt durch das Zwischenwirbelimplantat nach Fig. 5; und
Fig. 7 einen horizontalen Querschnitt durch das Zwischenwirbelimplantat nach Fig. 5.

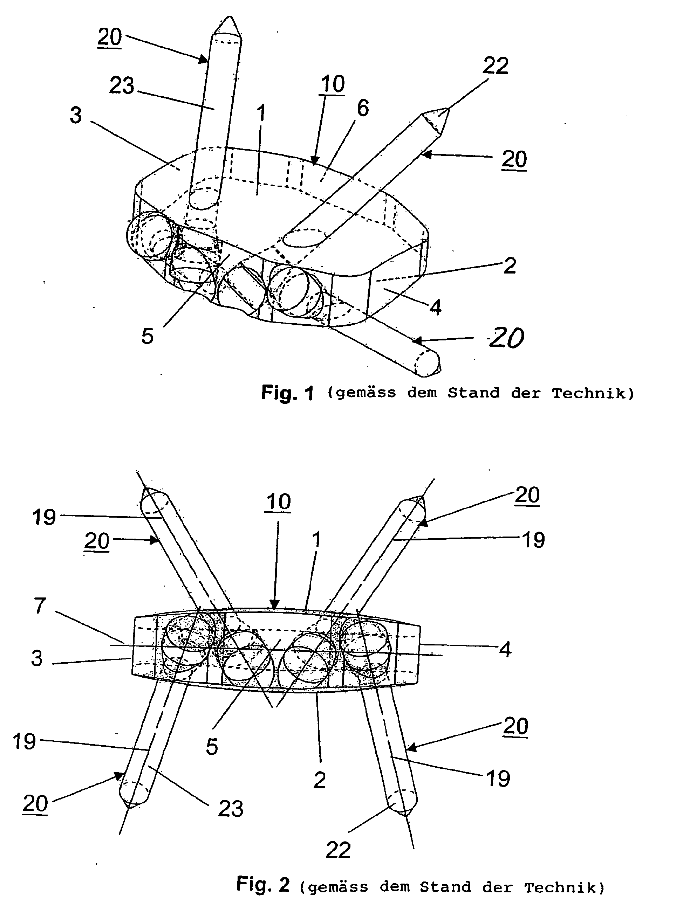

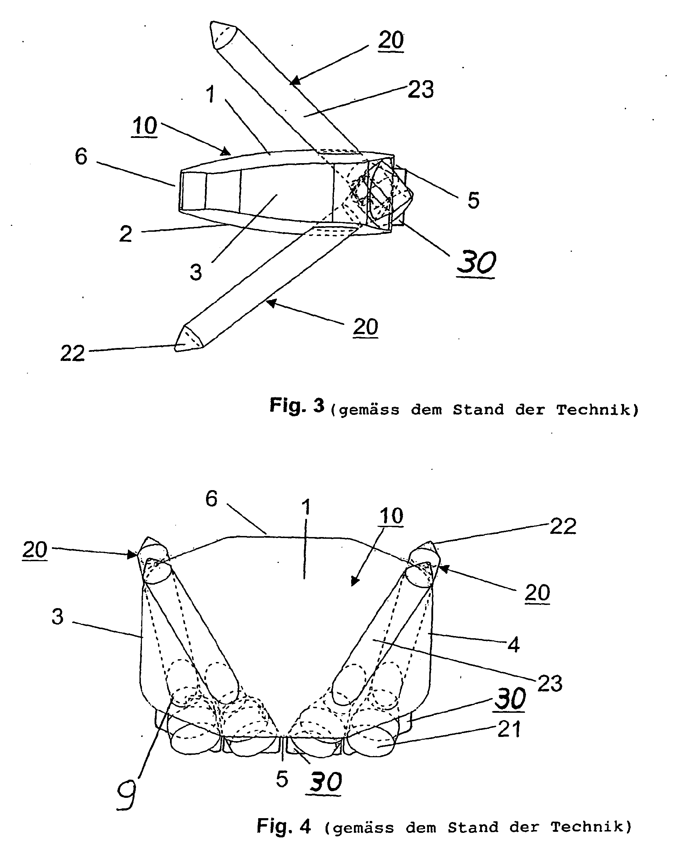

Das in den Fig. 1 bis 4 dargestellte, aus dem Stand der Technik bekannte Zwischenwirbelimplantat besteht aus einem dreidimensionalen Körper 10 mit einer konvex gewölbten Oberseite 1 und einer ebenfalls konvex gewölbten Unterseite 2, welche beide zur Anlage an die Endplatten zweier benachbarter Wirbelkörper bestimmt sind. Zur besseren Verankerung können die Oberseite 1 und Unterseite 2 strukturiert sein, und mit Rillen, Rippen oder Zähnen versehen sein oder auch nur eine aufgerauhte Oberfläche aufweisen.
Der dreidimensionalen Körper 10 weist weiter eine linke Seitenfläche 3 und eine rechte Seitenfläche 4 auf sowie eine Vorderfläche 5 und eine Hinterfläche 6. Der Körper 10 kann auch hohl ausgebildet sein und dessen Mantelfläche kann mit Perforationen versehen sein.
Der Körper 10 ist mit einer Mehrzahl den Körper 10 durch dringenden Bohrungen 9 versehen, die zur Aufnahme von longitudinalen Fixationselementen 20 geeignet sind. Vorzugsweise sind vier solcher Bohrungen 9 vorhanden.
Die Achsen 19 der Bohrungen 9 schneiden die horizontale Mittelebene 7, welche zwischen der Oberseite 1 und der Unterseite 2 angeordnet ist.
Mindestens eine der Bohrungen 9 ist derart ausgebildet, dass ein darin aufgenommenes, longitudinales Fixationselement 20 in rigider Weise mit dem Zwischenwirbelimplantat verbindbar ist. Zu diesem Zweck sind die Bohrungen 9 konisch ausgebildet.

Die Fixationselemente 20 sind vorzugsweise Knochenschrauben mit einem Kopf 21 und einer Spitze 22. Der Kopf 21 verjüngt sich gegen den Schaft 23 hin konisch, wobei die Konizität des Kopfes 21 der Konizität der Bohrung 9 entspricht. Die vier Bohrungen 9 können zudem ein Innengewinde 11 aufweisen.

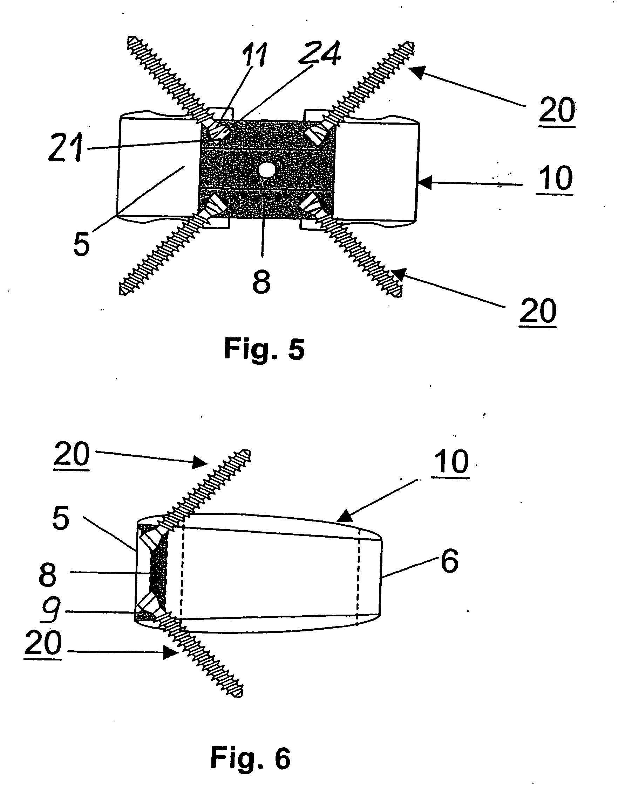

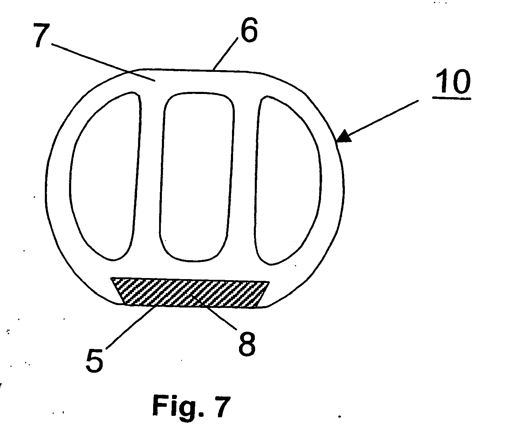

Bei der in den Fig. 5 - 7 erfindungsgemässen dargestellten Ausführung besitzt der dreidimensionalen Körper 10 an seiner Vorderfläche 5 einen vorzugsweise metallischen Einsatz 8 mit vier Bohrungen 9 zur Aufnahme der Fixationselemente 20. Die Fixationselemente 20, welche als Knochenschrauben mit einem ein Aussengewinde 24 aufweisenden Kopf 21 und einem Schaft 23 ausgebildet sind, werden mit dem Aussengewinde 24 im Innengewinde 11 der Bohrung 9 verankert. Der Einsatz 8 ist dabei im dreidimensionalen Körper 10 vertikal verschieblich angeordnet.

## Patentansprüche

1. Zwischenwirbelimplantat in Form eines dreidimensionalen Körpers (10) mit
A) einer Oberseite (1) und einer Unterseite (2), welche zur Anlage an die Endplatten zweier benachbarter Wirbelkörper geeignet sind;
B) einer linken Seitenfläche (3) und einer rechten Seitenfläche (4);
C) einer Vorderfläche (5) und einer Hinterfläche (6);
D) einer zwischen der Oberseite (1) und Unterseite (2) liegenden, horizontalen Mittelebene (7); sowie
E) einer Mehrzahl den Körper (10) durchdringenden Bohrungen (9), die zur Aufnahme von longitudinalen Fixationselementen (20) geeignet sind und deren Achsen (19) die horizontale Mittelebene (7) schneiden; wobei
F) mindestens eine der Bohrungen (9) derart ausgebildet ist, dass ein darin aufgenommenes, longitudinales Fixationselement (20) in rigider Weise mit dem Zwischenwirbelimplantat verbindbar ist,
**dadurch gekennzeichnet, dass**
G) der dreidimensionale Körper (10) an seiner Vorderfläche (5) eine zur horizontalen Mittelebene (7) vertikal angeordnete, als Einsatz ausgebildete Frontplatte (8) aufweist, durch welche die Bohrungen (9) hindurchgehen und in welcher die longitudinalen Fixationselemente (20) verankerbar sind

2. Zwischenwirbelimplantat nach Anspruch 1, **dadurch gekennzeichnet, dass** die Frontplatte (8) im dreidimensionalen Körper (10) vertikal zur Mittelebene (7) verschieblich angeordnet ist.

3. Zwischenwirbelimplantat nach Anspruch 1 oder 2, **dadurch gekennzeichnet, dass** die Frontplatte (8) aus einem anderen Material gefertigt ist als der dreidimensionale Körper (10).

4. Zwischenwirbelimplantat nach Anspruch 3, **dadurch gekennzeichnet, dass** die Frontplatte (8) metallisch ist.

5. Zwischenwirbelimplantat nach einem der Ansprüche 1 bis 4, **dadurch gekennzeichnet, dass** mindestens eine der Bohrungen (9) ein Innengewinde (11) aufweist.

6. Zwischenwirbelimplantat nach einem der Ansprüche 1 bis 5, **dadurch gekennzeichnet, dass** sich die mindestens eine Bohrung (9) gegen die Unterseite (2) hin konusförmig verjüngt.

7. Zwischenwirbelimplantat nach Anspruch 6, **dadurch gekennzeichnet, dass** die konische Bohrung (9) einen Konuswinkel besitzt, welcher kleiner als der resultierende Reibungswinkel ist.

8. Zwischenwirbelimplantat nach Anspruch 7, **dadurch gekennzeichnet, dass** die Konizität der konischen Bohrung (9) im Bereich von 1 : 3,75 bis 1 : 20,00, vorzugsweise im Bereich von 1 : 5 bis 1: 15 liegt.

9. Zwischenwirbelimplantat nach einem der Ansprüche 1 bis 8, **dadurch gekennzeichnet, dass** seine Seitenflächen (1,2,3,4,5,6) im wesentlichen alle konvex ausgebildet sind.

10. Zwischenwirbelimplantat nach einem der Ansprüche 1 bis 9, **dadurch gekennzeichnet, dass** die Oberseite (1) und/oder die Unterseite (2) nicht planar und vorzugsweise konvex ausgebildet ist.

11. Zwischenwirbelimplantat nach einem der Ansprüche 1 bis 10, **dadurch gekennzeichnet, dass** die Bohrungen (9) die linke Seitenfläche (3) und die rechte Seitenfläche (4) nicht durchbohren.

12. Zwischenwirbelimplantat nach einem der Ansprüche 1 bis 11, **dadurch gekennzeichnet, dass** die Bohrungen (9) die Vorderfläche (5) nicht durchbohren.

13. Zwischenwirbelimplantat nach einem der Ansprüche 1 bis 12, **dadurch gekennzeichnet, dass** mindestens zwei der Bohrungen (9) parallel verlaufen.

14. Zwischenwirbelimplantat nach einem der Ansprüche 1 bis 13, **dadurch gekennzeichnet, dass** mindestens zwei der Bohrungen (9) von der Vorderseite (1) aus betrachtet divergent verlaufen.

15. Zwischenwirbelimplantat nach einem der Ansprüche 1 bis 14, **dadurch gekennzeichnet, dass** die Achsen (19) der Bohrungen (9) zur horizontalen Mittelebene (7) einen Winkel im Bereich von 25° bis 70°, vorzugsweise von 35° bis 55°, einschliessen.

16. Zwischenwirbelimplantat nach einem der Ansprüche 1 bis 15, **dadurch gekennzeichnet, dass** die Bohrungen (9) die horizontale Mittelebene (7) nicht durchstossen.

17. Zwischenwirbelimplantat nach einem der Ansprüche 1 bis 16, mit mindestens zwei in den Bohrungen (9) eingeführten longitudinalen Fixationselemente (20) mit einem Kopf (21) und einer Spitze (22), **dadurch gekennzeichnet, dass** mindestens ein longitudinales Fixationselement (20) die Oberseite (1) und mindestens ein longitudinales Fixationselement (20) die Unterseite (2) durchstösst.

18. Zwischenwirbelimplantat nach Anspruch 17, **dadurch gekennzeichnet, dass** die longitudinalen Fixationselemente (20) als Knochenschrauben mit einem Kopf (21) und einem Schaft (23) ausgebildet sind.

19. Zwischenwirbelimplantat nach Anspruch 18, **dadurch gekennzeichnet, dass** der Kopf (21) mit einem Aussengewinde (24) versehen ist, welches mit dem Innengewinde (11) der Bohrung (9) korrespondiert.

20. Zwischenwirbelimplantat nach Anspruch 18 oder 19, **dadurch gekennzeichnet, dass** der Kopf (21) sich gegen den Schaft (23) hin konisch verjüngt, wobei vorzugsweise die Konizität des Kopfes (21) der Konizität der Bohrung (9) entspricht.

21. Zwischenwirbelimplantat nach einem der Ansprüche 17 bis 20, **dadurch gekennzeichnet, dass** mindestens zwei longitudinale Fixationselemente (20) die Oberseite (1) und mindestens zwei longitudinale Fixationselement (20) die Unterseite (2) durchstossen.

22. Zwischenwirbelimplantat nach einem der Ansprüche 17 bis 21, **dadurch gekennzeichnet, dass** die longitudinalen Fixationselemente (20) als Knochenschrauben ausgebildet sind, deren Schaft mit einem Aussengewinde (25) versehen ist, welches vorzugsweise selbstbohrend und selbstschneidend ist.

23. Zwischenwirbelimplantat nach einem der Ansprüche 17 bis 22, **dadurch gekennzeichnet, dass** die longitudinalen Fixationselemente (20) als gewindelose Zylinderstifte mit einer Bohrerspitze, vorzugsweise in Form eines Trokars, ausgebildet sind.

24. Zwischenwirbelimplantat nach einem der Ansprüche 17 bis 22, **dadurch gekennzeichnet, dass** die longitudinalen Fixationselemente (20) als Spiralfedern ausgebildet sind.

25. Zwischenwirbelimplantat nach einem der Ansprüche 17 bis 22, **dadurch gekennzeichnet, dass** die longitudinalen Fixationselemente (20) als ein- oder mehrflügelige Spiralklingen ausgebildet sind.

26. Zwischenwirbelimplantat nach einem der Ansprüche 17 bis 25, **dadurch gekennzeichnet, dass** die Spitze (22) des longitudinalen Fixationselementes (20) im Körper (10) verankerbar ist, so dass der Kopf (21) des longitudinalen Fixationselementes (20) im benachbarten Wirbelkörper verankerbar ist.

27. Zwischenwirbelimplantat nach Anspruch 26, **dadurch gekennzeichnet, dass** der Kopf (21) des longitudinalen Fixationselementes (20) einen erweiterten Durchmesser aufweist und dass zusätzlich eine Unterlagsscheibe (30) für diesen Kopf (21) vorgesehen ist, welche zur Anlage an den Wirbelkörper geeignet ist.

28. Zwischenwirbelimplantat nach einem der Ansprüche 17 bis 27, **dadurch gekennzeichnet, dass** der Körper (10) aus Kunststoff, vorzugsweise einem unverstärkten Kunststoff besteht.

29. Zwischenwirbelimplantat nach Anspruch 28, **dadurch gekennzeichnet, dass** das Aussengewinde (25) der Knochenschrauben (20) einen Lastflankenwinkel im Bereich von 11° bis 14°, vorzugsweise von 12° bis 13° aufweist.

30. Zwischenwirbelimplantat nach einem der Ansprüche 17 bis 29, **dadurch gekennzeichnet, dass** das Aussengewinde (25) der Knochenschrauben einen Steigungswinkel im Bereich von 6° bis 10°, vorzugsweise von 7° bis 9° aufweist.

31. Zwischenwirbelimplantat nach einem der Ansprüche 1 bis 30, **dadurch gekennzeichnet, dass** die Bohrung (9) eine Metallhülse mit Innengewinde (11) aufweist.

32. Zwischenwirbelimplantat nach einem der Ansprüche 1 bis 31, **dadurch gekennzeichnet, dass** es teilweise aus Kunststoff und - im Bereich der Bohrungen (9) aus Metall besteht.

33. Zwischenwirbelimplantat nach einem der Ansprüche 28 bis 32, **dadurch gekennzeichnet, dass** die Bohrungen (9) eine glatte Innenwand aufweisen, in welche der Gewindekopf eines metallischen, longitudinalen Fixationselementes (20) eingeschnitten oder eingeformt werden kann.

34. Zwischenwirbelimplantat nach einem der Ansprüche 1 bis 33, **dadurch gekennzeichnet, dass** dessen Oberseite (1) und Unterseite (2) mit einer Strukturierung, vorzugsweise in Form von Zähnen, versehen ist.

35. Zwischenwirbelimplantat nach einem der Ansprüche 1 bis 34, **dadurch gekennzeichnet, dass** es als Hohlkörper ausgebildet ist, dessen Mantelflächen vorzugsweise mit Perforationen versehen sind.

## Claims

1. An intervertebral implant in the form of a three-dimensional body (10), comprising
(a) a top side (1) and an underside (2) which are appropriate to rest against the end plates of two adjacent vertebras,
(b) a left side face (3) and a right side face (4),
(c) a front face (5) and a rear face (6),
(d) a horizontal center plane (7) situated between the top side (1) and the underside (2), and
(e) a plurality of boreholes (9) passing through the body (10) apt to receive longitudinal affixation elements (20) and comprising axes (19) that intersect the horizontal center plane (7), further
(f) at least one of the boreholes (9) is designed so that a longitudinal affixation element (20) received in it can be connected rigidly to the intervertebral implant,
**characterized in that**
(g) the three-dimensional body (10) comprises at its front face (5) a front plate (8) which is arranged vertically to the horizontal center plane (7) and configured as an insert, the boreholes (9) passing through said plate (8) and the longitudinal affixation elements (20) being anchorable in it.

2. Intervertebral implant as claimed in claim 1, **characterized in that** the front plate (8) is configured in the three-dimensional body (10) to be vertically displaceable relative to the center plane (7).

3. Intervertebral implant as claimed in either of claims 1 and 2, **characterized in that** the front plate (8) is made of a material different from that of the three-dimensional body (10).

4. Intervertebral implant as claimed in claim 3, **characterized in that** the front plate (4) is metallic.

5. Intervertebral implant as claimed in one of claims 1 through 4, **characterized in that** at least one of the boreholes (9) comprises an inside thread.

6. Intervertebral implant as claimed in one of claims 1 through 5, **characterized in that** at least one borehole (9) tapers conically toward the underside (2).

7. Intervertebral implant as claimed in claim 6, **characterized in that** the cone angle of the conical borehole (9) is smaller than the resulting angle of friction.

8. Intervertebral implant as claimed in claim 7, **characterized in that** the conicity of the conical borehole (9) is in the range of 1:3.75 to 1:20.00, preferably in the range of 1:5 to 1:15.

9. Intervertebral implant as claimed in one of claims 1 through 8, **characterized in that** its side faces (1, 2, 3, 4, 5, 6) are all substantially convex.

10. Intervertebral implant as claimed in one of claims 1 through 9, **characterized in that** the top side (1) and/or the underside (2) are non-planar and preferably convex.

11. Intervertebral implant as claimed in one of claims 1 through 10, **characterized in that** the boreholes (9) do not pass through the left side face (3) nor through the right side face (4).

12. Intervertebral implant as claimed in one of claims 1 through 11, **characterized in that** the boreholes (9) do not pass through the front face (5).

13. Intervertebral implant as claimed in one of claims 1 through 12, **characterized in that** at least two of the boreholes (9) run mutually parallel.

14. Intervertebral implant as claimed in one of claims 1 through 13, **characterized in that** at least two of the boreholes (9) diverge from each other as seen from the front side (1).

15. Intervertebral implant as claimed in one of claims 1 through 4, **characterized in that** the axes (19) of the boreholes (9) subtend an angle in the range of 25° to 70°, preferably 35° to 55°, with the horizontal center plane.

16. Intervertebral implant as claimed in one of claims 1 through 15, **characterized in that** the boreholes (9) do not pass through the horizontal center plane (7).

17. Intervertebral implant as claimed in one of claims 1 through 16, comprising at least two longitudinal affixation elements (20) which are inserted into the boreholes (9) and which are fitted with a head (21) and a tip (22), **characterized in that** at least one longitudinal affixation element (20) passes through the top side (1) and at least one longitudinal affixation element (20) passes through the underside (2).

18. Intervertebral implant as claimed in claim 17, **characterized in that** the longitudinal affixation elements (20) are bone screws having a head (21) and a shank (23).

19. Intervertebral implant as claimed in claim 18, **characterized in that** the head (21) is fitted with an external thread (24) which matches the inner thread (11) of the borehole (9).

20. Intervertebral implant as claimed in either of claims 18 and 19, **characterized in that** the head (21) conically tapers toward the shank (23), the conicity of the head (21) preferably matching the conicity of the borehole (9).

21. Intervertebral implant as claimed in one of claims 17 to 20, **characterized in that** at least two longitudinal affixation elements (20) pass through the top side (1) and at least two longitudinal affixation elements (20) pass through the underside (2).

22. Intervertebral implant as claimed in one of claims 17 through 21, **characterized in that** the longitudinal affixation elements (20) are bone screws of which the shanks are fitted with external threads (25) that preferably shall be self-boring and self-tapping.

23. Intervertebral implant as claimed in one of claims 17 through 22, **characterized in that** the longitudinal fixation affixation elements (20) are unthreaded cylindrical pins having a drilling tip preferably in the form of a trocar.

24. Intervertebral implant as claimed in one of claims 17 through 22, **characterized in that** the longitudinal fixation elements (20) are spiral springs.

25. Intervertebral implant as claimed in one of claims 17 through 22, **characterized in that** the longitudinal affixation elements (20) are single wing or multi-wing spiral blades.

26. Intervertebral implant as claimed in one of claims 17 through 25, **characterized in that** the tip (22) of the longitudinal affixation element (20) can be anchored in the implant structure (10) in a manner that the head (21) of the longitudinal affixation element (20) can be anchored in the adjacent vertebra.

27. Intervertebral implant as claimed in claim 26, **characterized by** the head (21) of the longitudinal affixation element (20) comprising a widened diameter and additionally by a support disk (30) designed to rest against the vertebra and being provided for said head.

28. intervertebral implant as claimed in one of claims 17 through 27, **characterized in that** the implant structure (10) consists of a plastic, preferably a non-reinforced plastic.

29. Intervertebral implant as claimed in claim 28, **characterized in that** the external thread (25) of the bone screws (20) comprise a load bevel angle in the range of 11° to 14°, preferably of 12° to 13°.

30. Intervertebral implant as claimed in one of claims 17 through 29, **characterized in that** the external thread (25) of the bone screws exhibits an angular pitch in the range of 6° to 10°, preferably 7° to 9°.

31. Intervertebral implant as claimed in one of claims 1 through 30, **characterized in that** the borehole (9) comprises a metal bush with an inner thread (11).

32. Intervertebral implant as claimed in one of claims 1 through 31, **characterized in that** it is made partly of plastic and **in that** in the zone of the boreholes (9) it is made of metal.

33. Intervertebral implant as claimed in one of claims 28 through 32, **characterized in that** the boreholes (9) exhibit a smooth inner wall into which the threaded head of a metal, longitudinal affixation element (20) may be cut or integrated.

34. Intervertebral implant as claimed in one of claims 1 through 33, **characterized in that** its top side (1) and its underside (2) are shaped preferably in the form of teeth.

35. Intervertebral implant as claimed in one of claims 1 through 34, **characterized in that** it is in the form of a hollow structure of which the outside surfaces are preferably perforated.

## Revendications

1. Implant intervertébral sous la forme d'un corps tridimensionnel (10), comprenant
A) une face supérieure (1) et une face inférieure (2), qui sont appropriées pour l'appui sur les plateaux d'extrémité de deux corps vertébraux adjacents ;
B) une face latérale gauche (3) et une face latérale droite (4) ;
C) une face avant (5) et une face arrière (6) ;
D) un plan médian horizontal (7) situé entre la face supérieure (1) et la face inférieure (2) ; et
E) une pluralité d'alésages (9) traversant le corps (10) qui sont appropriés pour loger des éléments de fixation longitudinaux (20) et dont les axes (19) coupent le plan médian horizontal (7) ; dans lequel
F) au moins un des alésages (9) est conçu de telle sorte qu'un élément de fixation longitudinal (20) logé dans celui-ci peut être relié de manière rigide à l'implant intervertébral,
**caractérisé en ce que**
G) le corps tridimensionnel (10) présente, au niveau de sa face avant (5), une plaque frontale (8) disposée verticalement au plan médian horizontal (7), conçue sous forme de pièce rapportée, qui est traversée par les alésages (9) et dans laquelle les éléments de fixation longitudinaux (20) peuvent être ancrés.

2. Implant intervertébral selon la revendication 1, **caractérisé en ce que** la plaque frontale (8) située dans le corps tridimensionnel (10) est disposée de manière à pouvoir coulisser verticalement au plan médian (7).

3. Implant intervertébral selon la revendication 1 ou 2, **caractérisé en ce que** la plaque frontale (8) est faite d'un matériau différent de celui du corps tridimensionnel (10).

4. Implant intervertébral selon la revendication 3, **caractérisé en ce que** la plaque frontale (8) est métallique.

5. Implant intervertébral selon l'une quelconque des revendications 1 à 4, **caractérisé en ce qu'**au moins un des alésages (9) présente un taraudage (11).

6. Implant intervertébral selon l'une quelconque des revendications 1 à 5, **caractérisé en ce que** le au moins un alésage (9) rétrécit de manière conique vers la face inférieure (2).

7. Implant intervertébral selon la revendication 6, **caractérisé en ce que** l'alésage conique (9) possède un angle de conicité qui est plus petit que l'angle de frottement résultant.

8. Implant intervertébral selon la revendication 7, **caractérisé en ce que** la conicité de l'alésage conique (9) se situe dans la gamme de 1:3,75 à 1:20,00, de préférence dans la gamme de 1:5 à 1:15.

9. Implant intervertébral selon l'une quelconque des revendications 1 à 8, **caractérisé en ce que** ses faces latérales (1, 2, 3, 4, 5, 6) sont presque toutes conçues sous une forme convexe.

10. Implant intervertébral selon l'une quelconque des revendications 1 à 9, **caractérisé en ce que** la face supérieure (1) et/ou la face inférieure (2) n'est pas conçue de manière plane et est de préférence convexe.

11. Implant intervertébral selon l'une quelconque des revendications 1 à 10, **caractérisé en ce que** les alésages (9) ne traversent pas la face latérale gauche (3) et la face latérale droite (4).

12. Implant intervertébral selon l'une quelconque des revendications 1 à 11, **caractérisé en ce que** les alésages (9) ne traversent pas la face avant (5).

13. Implant intervertébral selon l'une quelconque des revendications 1 à 12, **caractérisé en ce qu'**au moins deux des alésages (9) s'étendent parallèlement.

14. Implant intervertébral selon l'une quelconque des revendications 1 à 13, **caractérisé en ce qu'**au moins deux des alésages (9) s'étendent de manière divergente, vu depuis la face avant (1).

15. Implant intervertébral selon l'une quelconque des revendications 1 à 14, **caractérisé en ce que** les axes (19) des alésages (9) forment, avec le plan médian horizontal (7), un angle dans la gamme de 25° à 70°, de préférence de 35° à 55°.

16. Implant intervertébral selon l'une quelconque des revendications 1 à 15, **caractérisé en ce que** les alésages (9) ne traversent pas le plan médian horizontal (7).

17. Implant intervertébral selon l'une quelconque des revendications 1 à 16, comprenant au moins deux éléments de fixation longitudinaux (20), insérés dans les alésages (9), présentant une tête (21) et une pointe (22), **caractérisé en ce qu'**au moins un élément de fixation longitudinal (20) traverse la face supérieure (1) et au moins un élément de fixation longitudinal (20) traverse la face inférieure (2).

18. Implant intervertébral selon la revendication 17, **caractérisé en ce que** les éléments de fixation longitudinaux (20) sont conçus sous la forme de vis à os présentant une tête (21) et une tige (23).

19. Implant intervertébral selon la revendication 18, **caractérisé en ce que** la tête (21) est pourvue d'un filetage (24) qui correspond au taraudage (11) de l'alésage (9).

20. Implant intervertébral selon la revendication 18 ou 19, **caractérisé en ce que** la tête (21) rétrécit de manière conique vers la tige (23), la conicité de la tête (21) correspondant de préférence à la conicité de l'alésage (9).

21. Implant intervertébral selon l'une quelconque des revendications 17 à 20, **caractérisé en ce qu'**au moins deux éléments de fixation longitudinaux (20) traversent la face supérieure (1) et au moins deux éléments de fixation longitudinaux (20) traversent la face inférieure (2).

22. Implant intervertébral selon l'une quelconque des revendications 17 à 21, **caractérisé en ce que** les éléments de fixation longitudinaux (20) sont conçus sous forme de vis à os dont la tige est pourvue d'un filetage (25) qui est de préférence autoperceur et autotaraudeur.

23. Implant intervertébral selon l'une quelconque des revendications 17 à 22, **caractérisé en ce que** les éléments de fixation longitudinaux (20) sont conçus sous forme de goupilles cylindriques non filetées présentant une mèche, de préférence sous la forme d'un trocart.

24. Implant intervertébral selon l'une quelconque des revendications 17 à 22, **caractérisé en ce que** les éléments de fixation longitudinaux (20) sont conçus sous forme de ressorts hélicoïdaux.

25. Implant intervertébral selon l'une quelconque des revendications 17 à 22, **caractérisé en ce que** les éléments de fixation longitudinaux (20) sont conçus sous forme de lames hélicoïdales à une ou plusieurs pales.

26. Implant intervertébral selon l'une quelconque des revendications 17 à 25, **caractérisé en ce que** la pointe (22) de l'élément de fixation longitudinal (20) peut être ancrée dans le corps (10) de sorte que la tête (21) de l'élément de fixation longitudinal (20) peut être ancrée dans le corps vertébral adjacent.

27. Implant intervertébral selon la revendication 26, **caractérisé en ce que** la tête (21) de l'élément de fixation longitudinal (20) présente un diamètre élargi et **en ce qu'**une rondelle (30) est prévue en plus pour cette tête (21), rondelle qui est appropriée à l'appui sur le corps vertébral.

28. Implant intervertébral selon l'une quelconque des revendications 17 à 27, **caractérisé en ce que** le corps (10) est fait de matière plastique, de préférence d'une matière plastique non renforcée.

29. Implant intervertébral selon la revendication 28, **caractérisé en ce que** le filetage (25) des vis à os (20) présente un angle de flanc porteur dans la gamme de 11° à 14°, de préférence de 12° à 13°.

30. Implant intervertébral selon l'une quelconque des revendications 17 à 29, **caractérisé en ce que** le filetage (25) des vis à os présente un angle de pas dans la gamme de 6° à 10°, de préférence de 7° à 9°.

31. Implant intervertébral selon l'une quelconque des revendications 1 à 30, **caractérisé en ce que** l'alésage (9) présente une douille métallique pourvue d'un taraudage (11).

32. Implant intervertébral selon l'une quelconque des revendications 1 à 31, **caractérisé en ce qu'**il est fait en partie de matière plastique et, dans la zone des alésages (9), de métal.

33. Implant intervertébral selon l'une quelconque des revendications 28 à 32, **caractérisé en ce que** les alésages (9) présentent une paroi interne lisse dans laquelle la tête filetée d'un élément de fixation longitudinal métallique (20) peut être découpée ou moulée.

34. Implant intervertébral selon l'une quelconque des revendications 1 à 33, **caractérisé en ce que** sa face supérieure (1) et sa face inférieure (2) sont pourvues d'une texturation, de préférence sous la forme de dents.

35. Implant intervertébral selon l'une quelconque des revendications 1 à 34, **caractérisé en ce qu'**il est conçu sous forme de corps creux dont les surfaces d'enveloppe externes sont de préférence pourvues de perforations.
